# EUROPEAN PATENT APPLICATION

(11) **EP 4 616 867 A1**
(43) Date of publication of application: **17.09.2025**
(21) Application number: 23887962.1
(22) Date of filing: 06.11.2023
(51) Int. Cl.: A61K 45/06, A61K 31/4025, A61K 31/573, A61P 13/12

(54) **COMBINATION OF ENDOTHELIN RECEPTOR ANTAGONIST AND GLUCOCORTICOID FOR TREATING IGA NEPHROPATHY**

(30) Priority: 07.11.2022 CN 202211384729; 01.11.2023 CN 202311443896
(71) Applicant: Sanreno Therapeutics (Shanghai) Limited, Shanghai 200120 (CN)
(72) Inventor: HUANG, Haoyu, Shanghai 200120 (CN)
(74) Representative: Amodio, Eliana Lucia
(86) International application number: PCT/CN2023/129964
(87) International publication number: WO 2024/099272

(57) **Abstract**

The present invention relates to a method of treating IgA nephropathy, and to a pharmaceutical composition comprising an endothelin receptor antagonist and a glucocorticoid.

## Description

### TECHNICAL FIELD

The present invention relates to a pharmaceutical composition of an endothelin receptor antagonist and glucocorticoid for IgA nephropathy, and to a use of said endothelin receptor antagonist and glucocorticoid in preparing a pharmaceutical composition or kit for treating IgA nephropathy.

### BACKGROUND ART

IgA nephropathy (IgAN) is the most common primary glomerulonephritis in the world and a leading cause of chronic kidney disease and end-stage renal disease (ESRD). 15% to 20% of patients with IgA nephropathy develop end-stage kidney disease (ESKD) within 10 years of onset, while 30% to 40% of patients will develop end-stage kidney disease (ESKD) within 20 years of onset, and the incidence of IgAN in China is particularly high. Pathologically, IgAN is characterized by the deposition of circulating immune complexes composed of galactose-deficient IgA1 and glycan-specific IgG antibodies, which trigger glomerular mesangial cell activation and an inflammatory response, culminating in renal tissue cell damage. IgG-Gd-IgA1 is deposited in the glomerular mesangial area and triggers an inflammatory cascade reaction, including local secretion of chemokines, cytokines and reactive oxygen species as well as complement activation, which can lead to glomerular mesangial cell proliferation, inflammatory cell recruitment, and crescent formation, and also damage proximal renal tubular epithelial cells, resulting in tubulointerstitial fibrosis. Patients present with a range of symptoms, often including microscopic or gross haematuria and proteinuria. Patients may also develop high blood pressure due to ongoing kidney damage. A significant number of patients will develop end-stage renal disease (ESRD) within 10-20 years of diagnosis of IgAN. During this period, in addition to declining kidney function, patients experience a variety of symptoms that significantly reduce their quality of life.

As recorded in patent WO 2021126977 A1, atrasentan is a selective endothelin A receptor (ETA) small molecule antagonist, and studies on its use in the treatment of immunoglobulin A nephropathy (IgAN) are currently being undertaken by Chinook Therapeutics.

Although the use of surrogate clinical endpoints has greatly accelerated the development of innovative drugs, the selection of study endpoints for kidney disease is problematic. It takes an average of 20 years for IgAN to develop into uremia, such that observation of drug effectiveness requires a large sample size and 20 years of follow-up. This is obviously unrealistic, but the emergence of surrogate endpoints can solve this problem to a certain extent. KDIGO guidelines recommend that reducing proteinuria to under 1g/d is a surrogate marker for improved renal outcomes in IgAN, and reduction to under 1g/d is a reasonable treatment target. Proteinuria levels are often used as surrogate endpoints in clinical studies and have already been widely incorporated into the design of research on new drugs for the treatment of IgA nephropathy. In December 2021, the FDA approved Calliditas Therapeutics' new drug TARPEYO^{®} (budesonide) for the treatment of IgA nephropathy, the first approval based on the surrogate clinical endpoint of proteinuria reduction.

### SUMMARY OF THE INVENTION

The object of the present invention is to overcome the limitations of drug treatment by providing a more effective combination therapy for treating IgA nephropathy as well as a corresponding pharmaceutical composition and kit.

In order to solve the technical problems of the present invention, the invention is implemented by way of the following technical solutions:

A method for treating IgA nephropathy in a subject in need thereof, comprising administering to said subject an effective amount of an endothelin receptor antagonist and a glucocorticoid, wherein said endothelin receptor antagonist and glucocorticoid may be administered as a single dosage form separately or sequentially.

In a preferred technical solution of the present invention, the endothelin receptor antagonist is selected from any one of tezosentan, sparsentan, bosentan, macitentan, ambrisentan, sitaxsentan, aprocitentan, and atrasentan or their pharmaceutically acceptable salts, or any combination thereof.

In a preferred technical solution of the present invention, the endothelin receptor antagonist is selected from atrasentan or a pharmaceutically acceptable salt thereof.

In a preferred technical solution of the present invention, the glucocorticoid is selected from any one of dexamethasone, betamethasone, fluorometholone, prednisone, prednisolone, methylprednisolone, hydrocortisone, fluocinolone, fluticasone, mometasone, loteprednol etabonate, rimexalone, fluticasone, beclomethasone, ciclesonide, budesonide, triamcinolone acetonide, prednicarbate, butixocort, tipredane and tixocortol or their pharmaceutically acceptable salts, or any combination thereof.

In a preferred technical solution of the present invention, the glucocorticoid is selected from budesonide and methylprednisolone or pharmaceutically acceptable salts thereof.

In a preferred technical solution of the present invention, the endothelin receptor antagonist is administered in a dosage form given orally, buccally, or parenterally. For example, the oral dosage form may be tablets, capsules, powders, pills, granules, suspensions, solutions and solution preconcentrates, or emulsions and emulsion preconcentrates, and the parenteral dosage form, for example, may be a dosage form administered intravenously, intraperitoneally, intradermally, subcutaneously, intramuscularly, intracranially, intrathecally, intratumorally, transdermally or transmucosally.

In a preferred technical solution of the present invention, the endothelin receptor antagonist is administered once or twice a day, or the endothelin receptor antagonist is administered once every 2, 3, 4, 5, 6, 7, 8, 9 or 10 days or once every 1, 2 or 3 weeks, or the endothelin receptor antagonist is administered once daily for 5 consecutive days per week, followed by an interval of 2 days.

In a preferred technical solution of the present invention, the endothelin receptor antagonist is administered to the subject in an effective dose of about 0.5 to about 250 mg/kg, 1 to about 250 mg/kg, about 2 to about 200 mg/kg, about 3 to about 120 mg/kg, about 5 to about 250 mg/kg, about 10 to about 200 mg/kg, or about 20 to about 120 mg/kg.

In a preferred technical solution of the present invention, the glucocorticoid is administered in a dosage form given orally, buccally or parenterally. For example, the oral dosage form may be tablets, capsules, powders, pills, granules, suspensions, solutions and solution preconcentrates, or emulsions and emulsion preconcentrates, and the parenteral dosage form, for example, may be a dosage form administered intravenously, intraperitoneally, intradermally, subcutaneously, intramuscularly, intracranially, intrathecally, intratumorally, transdermally or transmucosally.

In a preferred technical solution of the present invention, the glucocorticoid is administered once or twice a day, or the glucocorticoid is administered once every 2, 3, 4, 5, 6, 7, 8, 9 or 10 days or once every 1, 2 or 3 weeks, or the glucocorticoid is administered once daily for 5 consecutive days per week, followed by an interval of 2 days.

In a preferred technical solution of the present invention, the glucocorticoid is administered to the subject in an effective dose of about 0.5 to about 250 mg/kg, 1 to about 250 mg/kg, about 2 to about 200 mg/kg, about 3 to about 120 mg/kg, about 5 to about 250 mg/kg, about 10 to about 200 mg/kg, or about 20 to about 120 mg/kg.

In a preferred embodiment of the present invention, the method may optionally further comprise administering to the subject an effective amount of a third therapeutically active substance.

In a preferred technical solution of the present invention, the third therapeutically active substance is selected from an SGLT-2 inhibitor.

In a preferred technical solution of the present invention, the SGLT-2 inhibitor is selected from any one of empagliflozin, canagliflozin, remogliflozin, ipragliflozin, HM41322, dapagliflozin, bexagliflozin, ertugliflozin, sotagliflozin, luseogliflozin and tofogliflozin, or any combination thereof.

In a preferred technical solution of the present invention, the SGLT-2 inhibitor is administered in a dosage form given orally, buccally or parenterally. For example, the oral dosage form may be tablets, capsules, powders, pills, granules, suspensions, solutions and solution preconcentrates, or emulsions and emulsion preconcentrates, and the parenteral dosage form, for example, may be a dosage form administered intravenously, intraperitoneally, intradermally, subcutaneously, intramuscularly, intracranially, intrathecally, intratumorally, transdermally or transmucosally.

In a preferred technical solution of the present invention, the SGLT-2 inhibitor is administered once or twice a day, or the SGLT-2 inhibitor is administered once every 2, 3, 4, 5, 6, 7, 8, 9 or 10 days or once every 1, 2 or 3 weeks, or the SGLT-2 inhibitor is administered once daily for 5 consecutive days per week, followed by an interval of 2 days.

In a preferred technical solution of the present invention, the SGLT-2 inhibitor is administered to the subject in an effective dose of about 0.5 to about 250 mg/kg, 1 to about 250 mg/kg, about 2 to about 200 mg/kg, about 3 to about 120 mg/kg, about 5 to about 250 mg/kg, about 10 to about 200 mg/kg, or about 20 to about 120 mg/kg.

In a preferred technical solution of the present invention, the third therapeutically active substance is selected from an APRIL neutralizing antibody.

In a preferred technical solution of the present invention, the APRIL neutralizing antibody is BION-1301 monoclonal antibody.

In a preferred technical solution of the present invention, the APRIL neutralizing antibody is formulated into a solution or lyophilized agent.

In a preferred technical solution of the present invention, when the APRIL neutralizing antibody is administered as an intravenous injection, when the pH is 5-8, preferably 5.5, the concentration of the buffer solution is 1-50 mM, preferably 5-40 mM, and more preferably 10-20 mM, wherein the buffer solution is preferably histidine buffer solution, the concentration of the APRIL neutralizing antibody is 10-60 mg/mL, preferably 20-50 mg/mL, and more preferably 25 mg/mL, the concentration of sucrose is 1-10% (w/v), preferably 2-8% (w/v), and the concentration of polysorbate 80 is 0.01%-1% (w/v), preferably 0.01%-1% (w/v).

In a preferred technical solution of the present invention, the APRIL neutralizing antibody is administered once every 1, 2, 3, 4, 5, 6, 7, 8, 9, or 10 days or once every 1, 2, 3, 4, 5, 6, 7, 8, 9 or 10 weeks, or the APRIL neutralizing antibody is administered once daily for 5 consecutive days a week, followed by an interval of 2 days.

In a preferred technical solution of the present invention, the APRIL neutralizing antibody is administered at an effective dose of 0.05 mg/kg, 0.1 mg/kg, 1 mg/kg, 2 mg/kg, 3 mg/kg, 4 mg/kg, 5 mg/kg, 6 mg/kg, 7 mg/kg or 8 mg kg.

In addition, the present invention further provides a pharmaceutical composition comprising an effective amount of an endothelin receptor antagonist and a glucocorticoid, wherein the endothelin receptor antagonist and glucocorticoid may be administered as a single dosage form separately or sequentially.

In a preferred technical solution of the present invention, the endothelin receptor antagonist can be formulated into a dosage form that is administered orally, buccally or parenterally. For example, the oral dosage form may be tablets, capsules, powders, pills, granules, suspensions, solutions and solution preconcentrates, or emulsions and emulsion preconcentrates, and the parenteral dosage form, for example, may be a dosage form administered intravenously, intraperitoneally, intradermally, subcutaneously, intramuscularly, intracranially, intrathecally, intratumorally, transdermally or transmucosally, such as solutions or lyophilized agents; and/or

the glucocorticoid can be formulated into a dosage form that is administered by a parenteral route, for example, intravenously, intraperitoneally, intradermally, subcutaneously, intramuscularly, intracranially, intrathecally, intratumorally, transdermally or transmucosally, and may, for example, be a solution or a lyophilized agent.

In a preferred technical solution of the present invention, the endothelin receptor antagonist is selected from any one of tezosentan, sparsentan, bosentan, macitentan, ambrisentan, sitaxsentan, aprocitentan, and atrasentan or their pharmaceutically acceptable salts, or any combination thereof.

In a preferred technical solution of the present invention, the endothelin receptor antagonist is selected from atrasentan or a pharmaceutically acceptable salt thereof.

In a preferred technical solution of the present invention, the glucocorticoid is selected from any one of dexamethasone, betamethasone, fluorometholone, prednisone, prednisolone, methylprednisolone, hydrocortisone, fluocinolone, fluticasone, mometasone, loteprednol etabonate, rimexalone, fluticasone, beclomethasone, ciclesonide, budesonide, triamcinolone acetonide, prednicarbate, butixocort, tipredane and tixocortol or their pharmaceutically acceptable salts, or any combination thereof.

In a preferred technical solution of the present invention, the glucocorticoid is selected from budesonide and methylprednisolone or pharmaceutically acceptable salts thereof.

In a preferred technical solution of the present invention, each unit dosage form contains a dose of 1-1000 mg of endothelin receptor antagonist, such as 1, 2, 3, 4, 5, 6, 7, 8, 9, 10, 11, 12, 13, 14, 15, 16, 17, 18, 19, 20, 25, 30, 35, 40, 50, 60, 70, 75, 80, 90, 100, 110, 120, 125, 130, 140, 150, 160, 170, 175, 180, 190, 200, 250, 300, 350, 400, 450, 500, 600, 700, 750, 800, 900, 1000 mg or a value between any two of the above values.

In a preferred technical solution of the present invention, each unit dosage form contains a dose of 1-5000 mg of glucocorticoid, such as 1, 2, 3, 4, 5, 6, 7, 8, 9, 10, 11, 12, 13, 14, 15, 16, 17, 18, 19, 20, 25, 30, 35, 40, 50, 60, 70, 75, 80, 90, 100, 110, 120, 125, 130, 140, 150, 160, 170, 175, 180, 190, 200, 250, 300, 350, 400, 450, 500, 600, 700, 750, 800, 900, 1000, 1100, 1200, 1300, 1400, 1500, 1600, 1700, 1800, 1900, 2000, 2200, 2400, 2500, 2600, 2700, 2750, 2800, 3000, 3500, 4000, 4500, 5000 mg or a value between any two of the above values.

In a preferred technical solution of the present invention, the pharmaceutical composition may also optionally contain an effective amount of a third therapeutically active substance.

In a preferred technical solution of the present invention, the third therapeutically active substance is selected from an SGLT-2 inhibitor.

In a preferred technical solution of the present invention, the SGLT-2 inhibitor is selected from any one of empagliflozin, canagliflozin, remogliflozin, ipragliflozin, HM41322, dapagliflozin, bexagliflozin, ertugliflozin, sotagliflozin, luseogliflozin and tofogliflozin, or any combination thereof.

In a preferred technical solution of the present invention, the SGLT-2 inhibitor is selected from dapagliflozin.

In a preferred technical solution of the present invention, each unit dosage form contains a dose of 1-1000 mg of SGLT-2 inhibitor, such as 1, 2, 3, 4, 5, 6, 7, 8, 9, 10, 11, 12, 13, 14, 15, 16, 17, 18, 19, 20, 25, 30, 35, 40, 50, 60, 70, 75, 80, 90, 100, 110, 120, 125, 130, 140, 150, 160, 170, 175, 180, 190, 200, 250, 300, 350, 400, 450, 500, 600, 700, 750, 800, 900, 1000 mg or a value between any two of the above values.

In a preferred technical solution of the present invention, the third therapeutically active substance is selected from an APRIL neutralizing antibody.

In a preferred technical solution of the present invention, the APRIL neutralizing antibody is a BION-1301 monoclonal antibody.

In a preferred technical solution of the present invention, each unit dosage form contains a dose of 1-5000 mg of APRIL neutralizing antibody, such as 1, 2, 3, 4, 5, 6, 7, 8, 9, 10, 11, 12, 13, 14, 15, 16, 17, 18, 19, 20, 25, 30, 35, 40, 50, 60, 70, 75, 80, 90, 100, 110, 120, 125, 130, 140, 150, 160, 170, 175, 180, 190, 200, 250, 300, 350, 400, 450, 500, 600, 700, 750, 800, 900, 1000, 1100, 1200, 1300, 1400, 1500, 1600, 1700, 1800, 1900, 2000, 2200, 2400, 2500, 2600, 2700, 2750, 2800, 3000, 3500, 4000, 4500, 5000 mg or a value between any two of the above values.

In a preferred technical solution of the present invention, when the APRIL neutralizing antibody is administered as an intravenous injection, when the pH is 5-8, preferably 5.5, the concentration of the buffer solution is 1-50 mM, preferably 5-40 mM, and more preferably 10-20 mM, wherein the buffer solution is preferably histidine buffer solution, the concentration of the APRIL neutralizing antibody is 10-60 mg/mL, preferably 20-50 mg/mL, and more preferably 25 mg/mL, the concentration of sucrose is 1-10% (w/v), preferably 2-8% (w/v), and the concentration of polysorbate 80 is 0.01%-1% (w/v), preferably 0.01%-1% (w/v).

In addition, the present invention further provides a pharmaceutical kit, which contains any of the above pharmaceutical compositions of the present invention as well as instructions for use.

### DESCRIPTION OF THE DRAWINGS

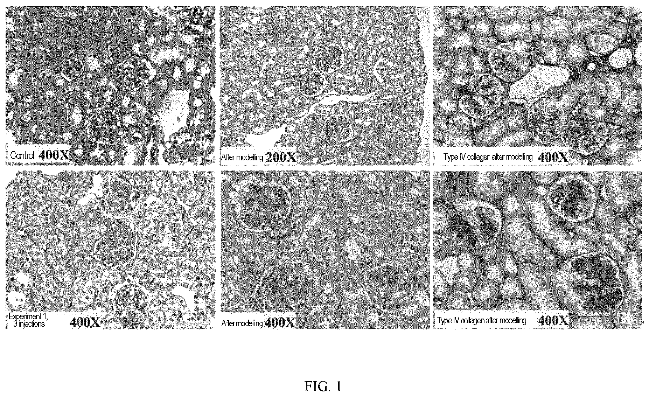

FIG. 1: Glomerular changes in mice before and after IgAN modelling;

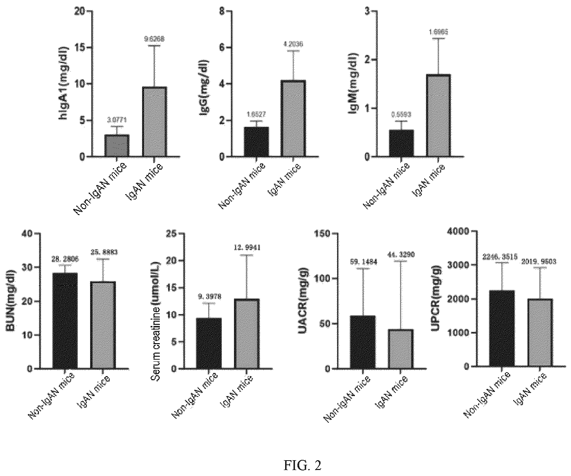

FIG. 2: Changes in mouse hIgA1, IgG, IgM, etc. before and after IgAN modelling;
FIG. 3-1, FIG. 3-2: Comparison of changes in hlgA1 index in IgAN mice administered a blank control (Ctrl), atrasentan (A), methylprednisolone (MP), a combination of atrasentan (A) + methylprednisolone (MP), or a combination of atrasentan (A) + methylprednisolone (MP) + dapagliflozin (Dapa);
FIG. 4-1, FIG. 4-2: Comparison of changes in IgG index in IgAN mice administered a blank control (Ctrl), atrasentan (A), methylprednisolone (MP), a combination of atrasentan (A) + methylprednisolone (MP), or a combination of atrasentan (A) + methylprednisolone (MP) + dapagliflozin (Dapa);
FIG. 5-1, FIG. 5-2: Comparison of changes in IgM index in IgAN mice administered a blank control (Ctrl), atrasentan (A), methylprednisolone (MP), a combination of atrasentan (A) + methylprednisolone (MP), or a combination of atrasentan (A) + methylprednisolone (MP) + dapagliflozin (Dapa).

### DETAILED DESCRIPTION OF THE PREFERRED EMBODIMENTS

### Definition of Terms:

Unless otherwise indicated herein, terms used herein have their conventional meanings in the art to which they belong.

"Treat" or "treatment" mean to reduce, inhibit, and/or reverse the progression of disease in a subject in need thereof. The term "treatment" includes any indication of successful treatment or improvement of a disease, including any objective or subjective parameter, such as alleviation, easing, reduction of symptoms, or making the injury, pathology or disorder more tolerable to the subject, or delaying or slowing the rate of progression, etc. Measurement of treatment or improvement may be based, for example, on the results of physical, pathological, and/or diagnostic examinations known in the art. For example, in one embodiment, "treating" IgA nephropathy as contemplated by the present invention means treating a subject with IgA nephropathy using a combination of an endothelin receptor antagonist and a glucocorticoid to achieve at least one positive therapeutic outcome.

Treatment may also refer to reducing the risk of a disease onset or episode, or reducing the recurrence of the disease (e.g. prolonging the time to recurrence) compared to what would occur if the measure was not taken. In the medical field, this treatment is also known as "prophylaxis".

The term "effective amount" or "therapeutically effective amount" refers to an amount effective in treating a disease as documented through clinical testing and evaluation, patient observation, and the like. "Effective amount" may further mean an amount that causes a detectable change in biological or chemical activity. Detectable changes can be detected and/or further quantified by one skilled in the art familiar with the relevant mechanisms or methods. Additionally, an "effective amount" may mean an amount that preserves a desired physiological state (i.e., reduces or prevents significant decline and/or promotes improvement of a condition). "Effective amount" may further refer to a therapeutically effective amount.

In some embodiments, the endothelin receptor antagonist is administered to the subject in an effective amount. The effective dose is usually 0.01 mg/kg body weight to 500 mg/kg body weight per day. In some embodiments, pharmaceutically acceptable compositions can be formulated such that 0.01 mg/kg to 200 mg/kg body weight or 0.01 mg/kg to 100 mg/kg body weight per day can be administered to a subject receiving these compositions (e.g. a dose of 0.75 mg to 7.5 g or 15 g for a 75 kg human). In certain embodiments, the active pharmaceutical ingredients of the present invention are formulated to provide a dose of 0.01 mg/kg to 70 mg/kg (e.g. a dose of 0.75 mg to 5.25 g for a 75 kg human).

In some embodiments, the effective dose of the endothelin receptor antagonist is about 0.5 to about 250 mg/kg, 1 to about 250 mg/kg, about 2 to about 200 mg/kg, about 3 to about 120 mg/kg, about 5 to about 250 mg/kg, about 10 to about 200 mg/kg, or about 20 to about 120 mg/kg. In some embodiments, effective doses include about 0.5 mg/kg, 1 mg/kg, 2 mg/kg, 3 mg/kg, 4 mg/kg, 5 mg/kg, 6 mg/kg, 8 mg/kg, 10 mg/kg, 20 mg/kg, 25 mg/kg, 40 mg/kg, 50 mg/kg, 60 mg/kg, 75 mg/kg, 100 mg/kg, 120 mg/kg, 150 mg/kg, 175 mg/kg, 200 mg/kg, 225 mg/kg, 250 mg/kg and 300 mg/kg. The dosage form may take a variety of suitable forms, such as a tablet or capsule, and the effective dose may be provided in one or more unit dosage forms (such as tablets or capsules) and administered 1, 2 or 3 times daily, or, for example, at 4, 8 or 12 hour intervals throughout the day. A tablet or capsule may contain, for example, 10, 25, 50, 75, 100, 150, 200, 250, 300, 350, 400, 450, 500, 600, 700, 800, 900, 1,000, 1,100 or 1,250 mg of compound. For example, in some embodiments, administration of an endothelin receptor antagonist to a human subject can include a daily dose of the endothelin receptor antagonist in the range of 100-1,250, 150-1,000, 200-800, or 250-750 mg. The daily dose may be administered in its entirety once a day, or in multiple portions administered at regular intervals throughout the day. Liquid formulations may also be prepared, allowing for easy and convenient dispensing of any dosage.

The antibodies will typically be mixed with a pharmaceutically acceptable non-toxic carrier material (e.g. physiological saline or phosphate buffered saline) prior to administration, and may be administered using any medically appropriate procedure, including, but not limited to, for example, intravenous or intraarterial administration and injection into the cerebrospinal fluid.

In some embodiments, an effective dose of the antibody is about 5 to about 250 mg/kg, about 10 to about 200 mg/kg, or about 20 to about 120 mg/kg. In some embodiments, effective doses include 5 mg/kg, 10 mg/kg, 20 mg/kg, 25 mg/kg, 40 mg/kg, 50 mg/kg, 60 mg/kg, 75 mg/kg, 100 mg/kg, 120 mg/kg, 150 mg/kg, 175 mg/kg, 200 mg/kg, 225 mg/kg, 250 mg/kg and 300 mg/kg. The dosage form may take the form of, for example, a tablet or capsule, and the effective dose may be provided in one or more tablets or capsules, etc., and may be provided once daily or at intervals throughout the day, for example, 4, 8 or 12 hours. A tablet or capsule may contain, for example, 10, 25, 50, 75, 100, 150, 200, 250, 300, 350, 400, 450, 500, 600, 700, 800, 900 or 1,000 mg of antibody. Liquid formulations may also be prepared, allowing for easy and convenient dispensing of any dosage.

In some embodiments, the antibody is administered to the subject in an effective amount. The effective dose is usually 0.01 mg/kg body weight to 500 mg/kg body weight per day. In some embodiments, pharmaceutically acceptable compositions can be formulated such that 0.01 mg/kg to 200 mg/kg body weight or 0.01 mg/kg to 100 mg/kg body weight per day can be administered to patients receiving these compositions (e.g. a dose of 0.75 mg to 7.5 g or 15 g for a 75 kg human. In certain embodiments, compositions of the present invention are formulated to provide a dose of 0.01 mg/kg to 70 mg/kg (e.g. a dose of 0.75 mg to 5.25 g for a 75 kg human).

An effective amount of antibody can be, for example, 0.05 mg/kg, 0.1 mg/kg, 1 mg/kg, 2 mg/kg, 3 mg/kg, 4 mg/kg, 5 mg/kg, 6 mg/kg, 7 mg/kg or 8 mg/kg per dose (e.g. a dose of 3.75 mg to 600 mg for a 75 kg human).

Doses of the antibody may be administered once, twice, three times, four times, five times or more times per week, once per week, once every two weeks, or even once every three weeks during the course of treatment. Dosing may be once daily, once every two days, once every three days, once every four days, once every five days, once a week, once every two weeks, or once every three weeks. Formulations containing antibodies can be prepared that allow any dosage to be dispensed easily and conveniently.

The term "subject" refers to a mammalian subject, and in particular a human subject, including male or female subjects, and includes newborn, infant, toddler, adolescent, adult or geriatric subjects, and further includes various human races and ethnicities, e.g. Caucasian, African and Asian races. Herein, the subject suffers from IgA nephropathy.

The term "pharmaceutically acceptable salts" refers to relatively non-toxic inorganic or organic acid salts of compounds. These salts may be prepared in situ during the final isolation and purification of the compound, or by reacting the free form of the purified compound with a suitable organic or inorganic acid, respectively, and isolating the salt so formed. Representative acid salts include, but are not limited to, acetate, adipate, aspartate, benzoate, benzenesulfonate, bicarbonate/carbonate, bisulfate/sulfate, borate, dextrorotary camphorsulfonate, citrate, cyclosulfonate, ethanedisulfonate, ethanesulfonate, formate, fumarate, glucoheptonate, gluconate, glucuronate, hexafluorophosphate, hypobenrate, hydrochloride/chloride, hydrobromide/bromide, hydroiodide/iodide, isethionate, lactate, malate, maleate, malonate, methane sulfonate, methylsulfate, naphthylate, 2-naphthalenesulfonate, nicotinate, nitrate, orotate, oxalate, palmitate, pamoate, phosphate/hydrogen phosphate/dihydrogen phosphate, pyroglutamate, glycolate, stearate, succinate, tannate, tartrate, tosylate, trifluoroacetate and xinafoate salts. In one embodiment, the pharmaceutically acceptable salt is a hydrochloride/chloride salt.

As used herein, the term "pharmaceutically acceptable" refers to types generally accepted by those skilled in the pharmaceutical art. For example, pharmaceutically acceptable salts, pharmaceutically acceptable carriers, etc.

"Oral dosage form" refers to a pharmaceutical formulation prepared for administration to an individual by the oral route of administration. Examples of known oral dosage forms include, but are not limited to, tablets, capsules, powders, pills, granules, suspensions, solutions and solution preconcentrates, emulsions and emulsion preconcentrates, and the like. In some methods, powders, pills, granules, capsules and tablets may be coated with suitable polymers or conventional coating materials to achieve, for example, greater stability in the gastrointestinal tract or to achieve a desired release rate. In addition, the capsule shell of the powder, pill or granule is further coated. Tablets may be scored to facilitate administration.

When the endothelin receptor antagonist or glucocorticoid of the present invention is administered as an oral preparation, it is preferably film-coated. Suitable films are known in the art and are commercially available or can be manufactured according to known methods. Usually, the film coating material is a hydrophilic polymer, such as polyethylene glycol, polyvinylpyrrolidone, polyvinyl alcohol, hydroxypropyl cellulose, hydroxymethyl cellulose, hydroxypropyl methyl cellulose, and the like. Film coating composition ingredients may include conventional amounts of plasticizers such as polyethylene glycol, triethyl citrate, diethyl phthalate, propylene glycol, and glycerin, opacifiers such as titanium dioxide, and colorants such as iron oxides, aluminium lakes, and the like. Typically, the film coating material is applied, for example, in an amount providing the film coating in the range of 1% to 6% of the solid oral dosage form.

When the endothelin receptor antagonist of the present invention is administered as an oral preparation, it may optionally further comprise at least one pharmaceutically acceptable carrier used in medicines. "Pharmaceutically acceptable carrier" refers to any pharmaceutically inert material that is substantially inactive and forms a substantial part of the formulation. Examples of such carriers include, but are not limited to, diluents and fillers, disintegrants, glidants, binders, stabilizers, colorants, flavouring agents, and preservatives.

As diluents, examples of options include, but are not limited to: microcrystalline cellulose, mannitol, powdered sugar, compressible sugar, dextran, dextrin, dextrose, lactose, cellulose powder, sorbitol, sucrose and talc, or combinations thereof, wherein the diluent can be 5% to 90% of the total weight of the oral preparation, preferably 10% to 80%, 20%-70%, 30%-60%, or 40%-50%.

As disintegrants, examples of options include, but are not limited to: cellulose, alginates, gums, crosslinked polymers such as crosslinked polyvinylpyrrolidone or crospovidone, croscarmellose sodium, croscarmellose calcium, soybean polysaccharide, sodium starch glycolate, guar gum or any combination thereof.

In this case, the disintegrant may be present in an amount of about 1% to 15%, preferably 2% to 10%, based on the total weight of the oral preparation.

As binders, examples of options include, but are not limited to: starch, cellulose or derivatives thereof, such as microcrystalline cellulose, hydroxypropyl cellulose, hydroxyethyl cellulose and hydroxypropyl methylcellulose, sucrose, dextrose, corn syrup, polysaccharides, gelatin, or any combination thereof. In this case, the binder may be present in an amount of 0.01 to 10%, preferably 1% to 10%, based on the total weight of the preparation.

As glidants, examples of options include, but are not limited to: colloidal silica, magnesium trisilicate, cellulose powder, talc or combinations thereof. Glidants may be present in an amount from 0.1% to 10%, preferably from 0.1% to 0.5%, based on the total weight of the composition.

Unless stated otherwise, the terms "comprising" and "including" as used herein denote open-ended and non-limiting inclusion. For example, other therapeutic agents may also be included in addition to the pharmaceutical combinations formed by the endothelin receptor antagonist and the APRIL neutralizing antibody of the present invention, such as "biotherapeutic agents" and "chemotherapeutic agents".

The choice of dosage regimen for a combination therapy of the invention depends on factors such as: the serum or tissue turnover rate of the entity, the level of symptoms, overall immunogenicity, and the accessibility of the target cells, tissue, or organ in the individual being treated. Preferably, the dosage regimen maximizes the amount of each therapeutic agent delivered to the patient consistent with an acceptable level of side effects. Accordingly, the dosage and frequency of administration of each biotherapeutic and chemotherapeutic agent in combination therapy will depend in part on the specific therapeutic agent, the severity of the IgA nephropathy being treated, and patient characteristics.

Continuous administration is particularly useful when the therapeutic agents in combination therapy are administered in different dosage forms (e.g., one active agent is oral and the other active agent is a sterile solution) and/or different dosage regimens (e.g., the endothelin receptor antagonist is administered at least daily as the active ingredient, whereas the APRIL neutralizing antibody is administered less frequently, e.g., weekly, fortnightly, or every three weeks).

WO 2016110587 A1 describes the sequence and preparation of BION-1301 monoclonal antibody, which is incorporated by reference herein in its entirety. Here, the full-length heavy chain amino acid sequence, heavy chain variable region sequence and CDR sequences of BION-1301 respectively correspond to SEQ ID NO: 52, SEQ ID NO: 40, SEQ ID NO: 5, SEQ ID NO: 6 and SEQ ID NO:7 in this patent application, while the full-length light chain amino acid sequence, light chain variable region sequence and CDR sequences respectively correspond to SEQ ID NO: 50, SEQ ID NO: 30, SEQ ID NO: 8, SEQ ID NO: 9 and SEQ ID NO: 10.

The present invention will be further described below through examples, but the examples are not meant to limit the scope of the present invention in any way. For the measurement methods of various parameters in the examples, reference should be made to Wan F, Wang H, Wang M, Lü J, Zhao M, Zhang H. Sustained release of Lactobacillus casei cell wall extract can induce a continuous and stable IgA deposition model. J Pathol. 2022;257(3):262-273. doi:10.1002/path.5884, and Xie X, Li J, Liu P, et al. Chimeric Fusion between Clostridium Ramosum IgA Protease and IgG Fc Provides Long-Lasting Clearance of IgA Deposits in Mouse Models of IgA Nephropathy. J Am Soc Nephrol. 2022;33(5):918-935. doi:10.1681/ASN.2021030372. The above-mentioned documents are incorporated into this application in their entirety.

### Example 1: Construction of an IgAN animal model

### (a) Construction of a humanized IGHA1 mouse model;

(b) Construction of a renal IgA deposition model with high-level expression of IgA in serum. The mouse model of the present invention can effectively express human IgA1 protein, ensure the functionality of the human IgA1 molecule in mice, is more specific than the existing conventional construction methods, and is closer to the natural traits of human IgA1. In addition, the modelling method for the CFA stimulation model is simple and easy to perform, has a high success rate, good repeatability, and does minimal damage to the body. It provides an effective experimental animal platform for exploring the pathogenesis of IgA nephropathy and new treatment strategies.

### Procedure:

### I. Construction of a humanized IGHA1 mouse model (for specific methods, reference should be made to patent CN 115197942 A, the full text of which is incorporated into this application)

1. The entire human IGHA1 gene sequence was introduced into the IGHA gene region of C57BL/6Jju mice, and the heavy chain region of mouse IgA was replaced with the heavy chain region of human IgA1 to construct humanized IgA1 mice having an O-glycosylated hinge region.
2. After verifying the gene sequence of the first mouse, homozygous humanized IGHA1 mice were obtained through multiple generations of backcrossing and self-crossing.

### II. Construction of a renal IgA deposition model

1. Complete Freunds adjuvant (CFA) and phosphate buffer solution (PBS) were mixed thoroughly and emulsified at a ratio of 1:1 to obtain a CFA injection that was intraperitoneally injected into 8-week-old humanized IGHA1 mice constructed in step 1. Four times the mouse body weight (4w µl) was injected every 2 weeks, administering a total of 6 injections over a time period of about 2.5 months.
2. In this experiment, the 8-week-old humanized IgA1 mice underwent an induction period of 2 to 5 months after the final injection of CFA.

**Table 1**

| | Non-IgAN transgenic mice | | IgAN mice | | p-value |
|---|---|---|---|---|---|
| | Mean (SD) | Median (Q1 - Q3) | Mean (SD) | Median (Q1 - Q3) | |
| hIgA1 | 3.08(0.44) | 2.96 (2.11 - 4.12) | 9.59(0.95) | 7.58 (5.73 - 11.63) | <0.001(Non-parametric test) |
| IgG | 1.65(0.13) | 1.60 (1.37 - 1.91) | 4.26(0.29) | 3.76 (3.15 - 4.60) | <0.001(Non-parametric test) |
| IgM | 0.56(0.07) | 0.55 (0.40 - 0.75) | 1.68(0.11) | 1.45 (1.14 - 2.10) | <0.001 (Non-parametric test) |

The test results showed that the hIgA1, IgG, and IgM levels of IgAN mice after modelling were significantly higher than those of unstimulated humanized IGHA1 transgenic mice (non-IgAN transgenic mice).

### Example 2: Study on the efficacy and safety of atrasentan (A) combined with methylprednisolone (MP) and dapagliflozin (Dapa) for IgA nephropathy

Method: 41 IgA nephropathy model mice were randomly assigned to an atrasentan + methylprednisolone + dapagliflozin group (n=9), atrasentan + methylprednisolone group (n=8), methylprednisolone group (n=8), atrasentan group (n=8) and treatment control group (n=8). Each group received intraperitoneal injections of CFA injection to create a model. Upon model completion, atrasentan, methylprednisolone and/or dapagliflozin were administered as outlined below. The control group was administered an equal amount of solvent (an aqueous solution of 0.5% (w/v) methylcellulose and 0.5% (v/v) Tween 80) by gavage for 30 days.

Dosing method: atrasentan (15 mg/kg/day), methylprednisolone (5 mg/kg/day), dapagliflozin (1 mg/kg/day) were administered by gavage every morning for 30 days. When co-administered, the required medicines were mixed together in the appropriate proportion (the dose of each medicine was calculated according to mouse body weight) before administration by gavage.

Before commencing dosing, once a week during the dosing period, and on day 30 of dosing, the body weight/blood pressure of the mice were measured, and the levels of hlgA1, Gd-IgA1, IgA-containing complexes, IgG, IgM, C3, BUN, Scr, Hb, BNP and ET-1 in the blood were measured. Prior to dosing, once every 2 weeks during the dosing period, and on day 30 of dosing, urine creatinine, urine protein level, and albumin to creatinine ratio (ACR) were measured, and the mice checked for the presence of haematuria. Prior to dosing and on day 30 of dosing, renal tissue pathology was scored according to the Oxford classification system, IgA and C3 deposition in renal tissue was measured by immunofluorescence assay, and mice in each group were examined for cardiac pathology.

Features of the IgAN mouse model:
- IGHA1+/+ mice stimulated intraperitoneally with CFA:
- Elevated levels of IgA1, Gd-IgA1 and IgA1 complex in serum and intestinal mucus
- Appearance of IgAN renal pathological phenotype:
   - IgA1 and C3 co-deposited in the mesangial area, with a stronger degree of complement activation as compared with stimulated wild-type mice.
   - Proliferation of mesangial matrix, mesangial cells and endothelial cells, with some mice exhibiting severe lesions in the form of focal sclerosis and tubulointerstitial fibrosis.

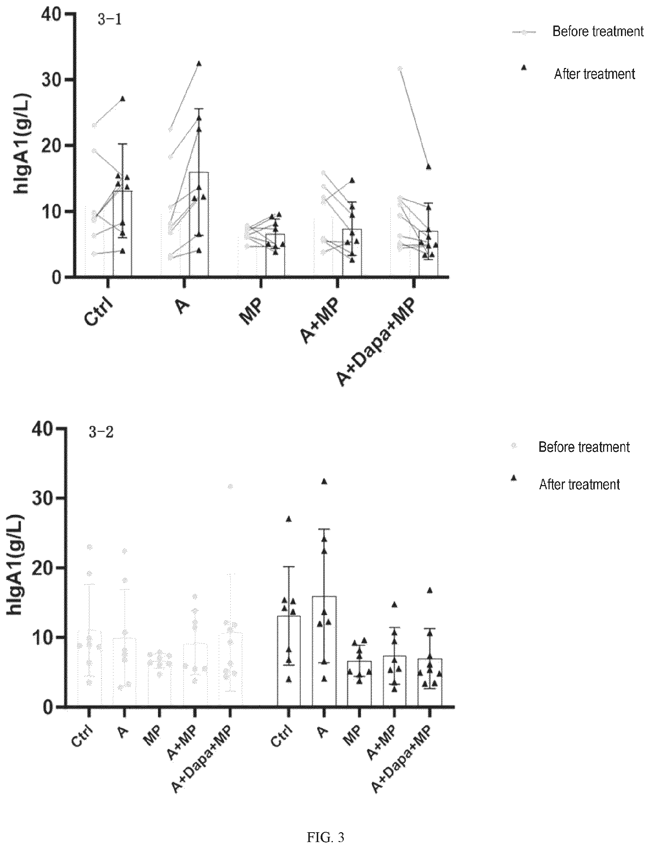

Analysing the test results, it can be seen from the data in Table 2 and FIG. 3-1 and FIG. 3-2 that, although there is no significant statistical difference between groups in hIgA1 changes, there is a trend for declining hIgA1 in the MP alone group as compared to the treatment control group (which exhibited an upward trend in hIgA1 after 30 days). This decline in hIgA1 after dosing was more pronounced in the A+MP group than in the MP alone group, and even more pronounced in the A+Dapa+MP group.

**Table 2**

| | Before treatment | | After treatment | | p-value | Difference between pre-treatment and post-treatment value (post-treatment minus pre-treatment) | |
|---|---|---|---|---|---|---|---|
| | Mean (SD) | Median (Q1 - Q3) | Mean (SD) | Median (Q1 - Q3) | | Mean (SD) | Median (Q1 - Q3) |
| Treatment control group (Ctrl) | 11.06 (2.34) | 8.89(6. 92 - 16.88) | 13.12( 2.51) | 14.00 (7.17 - 15.39) | 0.17 9 (Paired t test) | 2.05 (1.37) | 2.99 (-2.15 - 5.28) |
| Atrasentan (A) | 10.04 (2.46) | 7.88(4. 24 - 16.39) | 15.99( 3.40) | 12.99 (7.93 - 23.80) | 0.00 5 (Paired t test) | 5.95 (1.50) | 4.96 (3.03 - 8.99) |
| Methylprednisolone (MP) | 6.66( 0.37) | 6.69(6. 12 - 7.53) | 6.63(0 .79) | 6.23( 4.78 - 8.97) | 0.97 2 (Paired t test) | -0.03( 0.70) | -0.11 (-1.97 - 2.12) |
| Atrasentan + Methylprednisolone (A+MP) | 9.27( 1.63) | 8.68(5. 48 - 13.47) | 7.37(1 44) | 6.15( 4.05 - 10.44) | 0.14 4 (Paired t test) | -1.90 (1.16) | -2.60 (-4.98 - 1.26) |
| Atrasentan + Dapaglifiozin + Methylprednisolone (A+Dapa+MP) | 10.77 (2.82) | 9.36(5. 02 - 12.00) | 6.99(1 .43) | 5.36( 4.16 - 8.98) | 0.02 1 (Non-parametric test | -3.78( 1.63) | -1.70 (-6.11 - -0.41) |

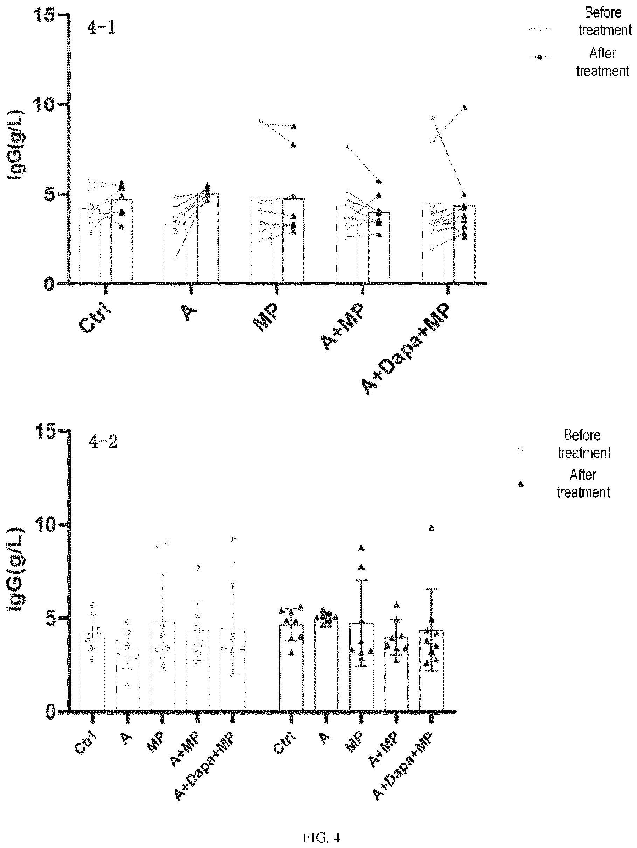

Analysing the test results, it can be seen from the data in Table 3 and FIG. 4-1 and FIG. 4-2 that, although there is no significant statistical difference between groups in IgG changes, there is a trend for declining IgG after dosing in the MP alone group as compared to the treatment control group (which exhibited an upward trend in IgG after 30 days), and this decline in IgG after dosing was more pronounced in the A+MP group than in the MP alone group.

**Table 3**

| | Before treatment | | After treatment | | p-value | Difference between pre-treatment and post-treatment value (post-treatment minus pre-treatment) | |
|---|---|---|---|---|---|---|---|
| | Mean (SD) | Median (Q1 - Q3) | Mean (SD) | Median (Q1 - Q3) | | Mean (SD) | Median (Q1 - Q3) |
| Treatment control group (Ctrl) | 4.23(0.33) | 4.08(3.57 - 5.09) | 4.67(0.31) | 4.90(3.94 - 5.43) | 0.249 | 0.45(0.36) | 0.39(-0.17 - 1.23) |
| Atrasentan (A) | 3.35(0.36) | 3.32(2.90 - 4.14) | 5.04(0.10) | 5.06(4.74 - 5.25) | 0.002 | 1.69(0.35) | 1.58(1.11 - 2.23) |
| Methylprednisolone (MP) | 4.85(0.94) | 3.75(3.04 - 7.83) | 4.75(0.81) | 3.56(3.22 - 7.06) | 1(Non-parametric test) | -0.10(0.20) | -0.09(-0.28 - 0.39) |
| Atrasentan + Methylprednisolone (A+MP) | 4.35(0.56) | 4.01(3.25 - 5.04) | 3.99(0.34) | 3.76(3.40 - 4.74) | 0.365 | -0.36(0.38) | -0.42(-1.15 - 0.34) |
| Atrasentan + Dapagliflozin + Methylprednisolone (A+Dapa+MP) | 4.49(0.82) | 3.45(3.07 - 6.14) | 4.38(0.73) | 3.79(3.01 - 4.66) | 0.441(Non-parametric test) | -0.11(0.61) | 0.45(-0.70 - 0.81) |

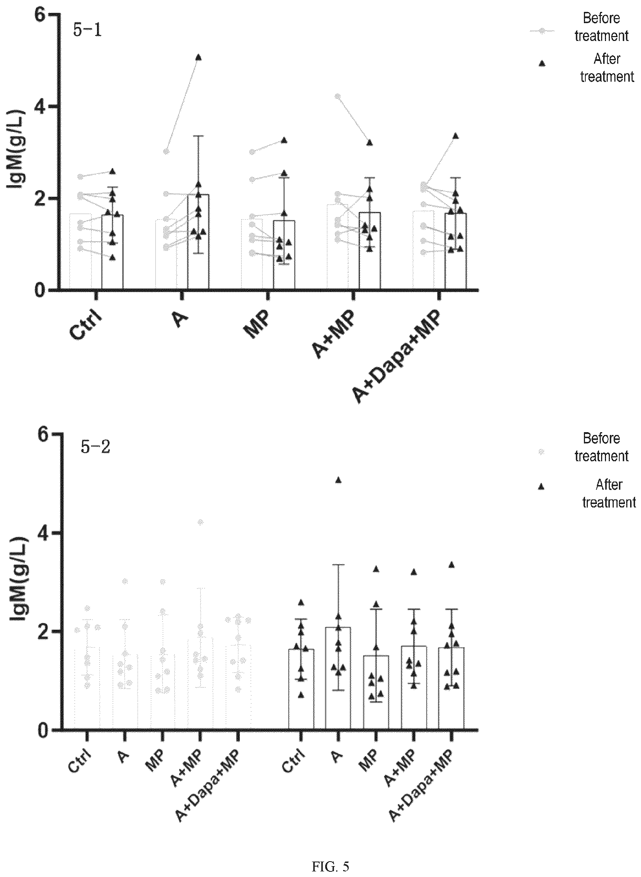

Analysing the test results (Table 4 and FIGs. 5-1 and 5-2), although there was no significant statistical difference between groups in IgM changes, the decline in IgM after dosing was more pronounced in the A+MP group as compared to the MP alone group (which displayed a mild downward trend in IgM after 30 days).

**Table 4**

| | Before treatment | | After treatment | | p-value | Difference between pre-treatment and post-treatment value (post-treatment minus pre-treatment) | |
|---|---|---|---|---|---|---|---|
| | Mean (SD) | Median (Q1 - Q3) | Mean (SD) | Median (Q1 -Q3) | | Mean (SD) | Median (Q1 - Q3) |
| Treatment control group (Ctrl) | 1.69(0.20) | 1.75(1.14 - 2.10) | 1.64(0.22) | 1.68(1.11 - 2.09) | 0.433 | -0.05(0.06) | -0.06(-0.17 - 0.10) |
| Atrasentan (A) | 1.55(0.25) | 1.31(1.02 - 1.97) | 2.08(0.45) | 1.72(1.28 - 2.26) | 0.025(Non-parametric test) | 0.54(0.23) | 0.38(0.07 - 0.69) |
| Methylprednisolone (MP) | 1.55(0.28) | 1.31(0.90 - 2.22) | 1.51(0.33) | 1.08(0.80 - 2.34) | 0.632 | -0.04(0.08) | -0.05(-0.12 - 0.13) |
| Atrasentan + Methylprednisolone (A+MP) | 1.88(0.36) | 1.49(1.28 - 2.07) | 1.70(0.27) | 1.39(1.20 - 2.16) | 0.263(Non-parametric test) | -0.18(0.18) | -0.15(-0.53 - 0.12) |
| Atrasentan + Dapaglifiozin + Methylprednisolone (A+Dapa+MP) | 1.73(0.19) | 1.88(1.24 - 2.24) | 1.68(0.26) | 1.72(1.04 - 2.04) | 0.74 | -0.06(0.16) | -0.17(-0.30--0.03) |

## Claims

1. Method of treating IgA nephropathy in a subject in need thereof, comprising administering to said subject an effective amount of an endothelin receptor antagonist and a glucocorticoid, wherein said endothelin receptor antagonist and glucocorticoid may be administered as a single dosage form separately or sequentially.

2. Method according to Claim 1, wherein the endothelin receptor antagonist is selected from any one of tezosentan, sparsentan, bosentan, macitentan, ambrisentan, sitaxsentan, aprocitentan and atrasentan or their pharmaceutically acceptable salts, or any combination thereof.

3. Method according to Claim 1 or 2, wherein the endothelin receptor antagonist is selected from atrasentan or a pharmaceutically acceptable salt thereof.

4. Method according to Claim 1, wherein the glucocorticoid is selected from any one of dexamethasone, betamethasone, fluorometholone, prednisone, prednisolone, methylprednisolone, hydrocortisone, fluocinolone, fluticasone, mometasone, loteprednol etabonate, rimexalone, fluticasone, beclomethasone, ciclesonide, budesonide, triamcinolone acetonide, prednicarbate, butixocort, tipredane and tixocortol or their pharmaceutically acceptable salts, or any combination thereof.

5. Method according to Claim 4, wherein the glucocorticoid is selected from any one of budesonide and methylprednisolone or pharmaceutically acceptable salts thereof.

6. Method according to Claim 1, wherein the endothelin receptor antagonist is administered in a dosage form given orally, buccally, or parenterally, such that the oral dosage form, for example, may be tablets, capsules, powders, pills, granules, suspensions, solutions and solution preconcentrates, or emulsions and emulsion preconcentrates, and the parenteral dosage form, for example, may be a dosage form administered intravenously, intraperitoneally, intradermally, subcutaneously, intramuscularly, intracranially, intrathecally, intratumorally, transdermally or transmucosally.

7. Method according to Claim 1, wherein the method optionally further comprises administering to the subject an effective amount of a third therapeutically active substance.

8. Method according to Claim 7, wherein the third therapeutically active substance is selected from an SGLT-2 inhibitor.

9. Method according to Claim 8, wherein the SGLT-2 inhibitor is selected from any one of empagliflozin, canagliflozin, remogliflozin, ipragliflozin, HM41322, dapagliflozin, bexagliflozin, ertugliflozin, sotagliflozin, luseogliflozin and tofogliflozin, or any combination thereof.

10. Pharmaceutical composition, comprising effective amounts of an endothelin receptor antagonist and a glucocorticoid, wherein said endothelin receptor antagonist and glucocorticoid can be administered as a single dosage form separately or sequentially.

11. Pharmaceutical composition according to Claim 10, wherein the endothelin receptor antagonist is selected from any one of tezosentan, sparsentan, bosentan, macitentan, ambrisentan, sitaxsentan, aprocitentan and atrasentan or their pharmaceutically acceptable salts, or any combination thereof.

12. Pharmaceutical composition according to Claim 10, wherein the endothelin receptor antagonist is selected from atrasentan or a pharmaceutically acceptable salt thereof.

13. Pharmaceutical composition according to Claim 10, wherein the glucocorticoid is selected from any one of dexamethasone, betamethasone, fluorometholone, prednisone, prednisolone, methylprednisolone, hydrocortisone, fluocinolone, fluticasone, mometasone, loteprednol etabonate, rimexalone, fluticasone, beclomethasone, ciclesonide, budesonide, triamcinolone acetonide, prednicarbate, butixocort, tipredane and tixocortol or their pharmaceutically acceptable salts, or any combination thereof.

14. Pharmaceutical composition according to Claim 13, wherein the glucocorticoid is selected from any one of budesonide and methylprednisolone or pharmaceutically acceptable salts thereof.

15. Pharmaceutical composition according to Claim 10, wherein the pharmaceutical composition may optionally further comprise an effective amount of a third therapeutically active substance.

16. Pharmaceutical composition according to Claim 15, wherein the third therapeutically active substance is selected from an SGLT-2 inhibitor.

17. Pharmaceutical composition according to Claim 16, wherein the SGLT-2 inhibitor is selected from any one of empagliflozin, canagliflozin, remogliflozin, ipragliflozin, HM41322, dapagliflozin, bexagliflozin, ertugliflozin, sotagliflozin, luseogliflozin and tofogliflozin, or any combination thereof.

18. Pharmaceutical composition according to Claim 17, wherein the SGLT-2 inhibitor is selected from dapagliflozin.
